(19) **Europäisches Patentamt**
**European Patent Office**
**Office européen des brevets**

(11) **EP 3 103 389 A1**

(12) **EUROPEAN PATENT APPLICATION**

(43) Date of publication:
**14.12.2016 Bulletin 2016/50**

(21) Application number: **16171530.5**

(22) Date of filing: **26.05.2016**

(51) Int Cl.:
*A61B 5/055* (2006.01)     *A61B 5/11* (2006.01)
*G01R 33/20* (2006.01)      *A61B 5/113* (2006.01)
*G01R 33/483* (2006.01)    *G01R 33/565* (2006.01)

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**MA MD**<br><br>(30) Priority: **27.05.2015  KR 20150073923**<br><br>(71) Applicants:<br> • **Samsung Electronics Co., Ltd.**<br>  **Gyeonggi-do 16677 (KR)** | • **Korea Advanced Institute of Science and Technology**<br>  **Daejeon 34141 (KR)**<br><br>(72) Inventors:<br> • **PARK, Hyun-wook**<br>  **Daejeon (KR)**<br> • **SEO, Hyun-seok**<br>  **Daejeon (KR)**<br><br>(74) Representative: **Land, Addick Adrianus Gosling et al**<br>  **Arnold & Siedsma**<br>  **Bezuidenhoutseweg 57**<br>  **2594 AC Den Haag (NL)** |

(54) **MAGNETIC RESONANCE IMAGING APPARATUS AND METHOD**

(57)     A magnetic resonance imaging MRI) apparatus, including a signal transceiver; a controller configured to control the signal transceiver to apply a bipolar gradient magnetic field in an aortic direction, the aortic direction being a direction in which an aorta is disposed within an object; and an image processor configured to receive, from the signal transceiver, phase signal data obtained by applying the bipolar gradient magnetic field to the object, acquire motion data representing a first motion corresponding to a heartbeat of the object and a second motion corresponding to a respiration of the object based on the phase signal data, and acquire MRI data based on the motion data.

**EP 3 103 389 A1**

## Description

This application claims benefit from Korean Patent Application No. 10-2015-0073923, filed on May 27, 2015, in the Korean Intellectual Property Office, the disclosure of which is incorporated herein in its entirety by reference.

The present disclosure relates to magnetic resonance imaging (MRI) apparatuses and methods, and more particularly, to MRI apparatuses and methods of acquiring MR images by using motion information of an object.

A magnetic resonance imaging (MRI) apparatus uses a magnetic field to capture an image of a target object, and is widely used for the accurate diagnosis of diseases because it shows stereoscopic images of bones, lumbar discs, joints, nerve ligaments, etc. at desired angles. When medical images are acquired using an MRI apparatus, motion of an object may hamper acquisition of high quality images.

To obtain high quality magnetic resonance (MR) images, information about motion of an object at a time point when an MR image is acquired is required. For example, motion of an object may include motions caused by a heartbeat and breathing of the object.

To acquire information about the motion of the object, a separate device for measuring the heartbeat and respiration of the object may be attached to the object. For example, electrocardiogram (ECG) gating equipment may be used to obtain information about the motion of the object due to the heartbeat thereof. An air bladder attached to an abdomen of the object may be used to obtain information about the motion of the object due to respiration.

To obtain information about the motion of an object via a separate device, in addition to the time required to acquire an MR image, extra time may be required to attach the separate device to the object.

Provided are magnetic resonance imaging (MRI) apparatuses and methods which are capable of acquiring an MR image of an object while simultaneously obtaining motion information related to a heartbeat and respiration of the object based on an MR signal acquired by performing an MRI scan on the object.

Furthermore, provided are MRI apparatuses and methods which are capable of obtaining, based on an MR signal acquired by performing an MRI scan on an object, motion information related to a heartbeat of the object simultaneously with motion information related to respiration of the object.

Additional aspects will be set forth in part in the description which follows and, in part, will be apparent from the description, or may be learned by practice of the presented embodiments.

According to an aspect of an exemplary embodiment, a magnetic resonance imaging (MRI) apparatus includes a signal transceiver; a controller configured to control the signal transceiver to apply a gradient magnetic field in a blood flow direction, the blood flow direction being a direction in which a blood vessel is disposed within an object ; and an image processor configured to receive, from the signal transceiver, phase signal data obtained by applying the gradient magnetic field to the object, acquire motion data representing a first motion corresponding to a heartbeat of the object based on the phase signal data, and acquire MRI data based on the motion data.

The gradient magnetic field may be a bipolar gradient magnetic field.

The image processor may be further configured to acquire motion data representing a second motion corresponding to a respiration of the object based on the phase signal data.

The image processor may be further configured to acquire projection data with respect to a first cross-section showing the aorta and a boundary of an abdomen of the object and to acquire the phase signal data based on the projection data.

The image processor may be further configured to acquire the MRI data with respect to a second cross-section different from the first cross-section.

The image processor may be further configured to acquire first motion data representing the first motion based on first phase signal data corresponding to the aorta, and to acquire second motion data representing the second motion based on second phase signal data corresponding to a boundary of an abdomen.

The image processor may be further configured to acquire the MRI data during a time interval determined based on the first motion data and the second motion data.

The image processor may be further configured to acquire the MRI data using a first readout gradient and acquire the motion data using a second readout gradient, the second readout gradient being different from the first readout gradient.

The MRI apparatus may further include a display configured to display at least one from among a first image generated based on the motion data and a second image generated based on the MRI data.

The image processor may be further configured to apply a high-pass filter to the first motion data.

According to another aspect of an exemplary embodiment, a magnetic resonance imaging (MRI) apparatus includes an image processor, the image processor being configured to: acquire projection data with respect to a first cross-section, the first cross-section not showing an aorta or a boundary of an abdomen of an object, the aorta and the boundary of the abdomen overlapping each other, by applying a bipolar gradient magnetic field to the object, obtain phase signal data based on the projection data, acquire motion data representing a first motion corresponding to a heartbeat of the object and a second motion corresponding to a respiration of the object based on the phase signal data, and acquire MRI data based on the motion data.

The image processor may be further configured to acquire first motion data based on first phase signal data corresponding to the aorta, and acquire second motion data based on second phase signal data corresponding to a boundary of an abdomen of the object.

The image processor may be further configured to acquire the MRI data during a time interval determined based on the first motion data and the second motion data.

The image processor may be further configured to acquire the MRI data with respect to a second cross-section different from the first cross-section.

The MRI apparatus may further include a controller configured to control the bipolar gradient magnetic field to be applied in an aortic direction, the aortic direction being a direction in which the aorta is disposed within the object.

The MRI apparatus may further include a display configured to display at least one from among a first image generated based on the motion data and a second image generated based on the MRI data.

According to a further aspect of an exemplary embodiment, a method of processing a magnetic resonance (MR) image includes controlling a gradient magnetic field to be applied in an blood flow direction, the blood flow direction being a direction in which a blood vessel is disposed within an object; acquiring, based on phase signal data obtained by applying the gradient magnetic field to the object, motion data representing a first motion corresponding to a heartbeat of the object; and acquiring magnetic resonance imaging (MRI) data based on the motion data.

The gradient magnetic field may be a bipolar gradient magnetic field.

The method may further include acquiring, based on phase signal data obtained by applying the gradient magnetic field to the abject, motion data representing a second motion corresponding to a respiration of the object.

The acquiring of the motion data may include: acquiring projection data with respect to a first cross-section showing the aorta and a boundary of an abdomen of the object by applying the bipolar gradient magnetic field to the object; and acquiring the phase signal data based on the projection data.

The acquiring of the MRI data may include acquiring, based on the motion data, the MRI data with respect to a second cross-section different from the first cross-section.

The acquiring of the motion data may include: acquiring the first motion data based on first phase signal data corresponding to the aorta; and acquiring the second motion data based on second phase signal data corresponding to the boundary of the abdomen.

The acquiring of the MRI data may include acquiring the MRI data during a time interval determined based on the first motion data and the second motion data.

The acquiring of the MRI data may include acquiring the MRI data by using a first readout gradient; and the acquiring of the motion data may include acquiring the motion data by using a second readout gradient different from the first readout gradient.

The method may further include displaying at least one from among a first image generated based on the motion data and a second image generated based on the MRI data.

The acquiring of the motion data may further include applying a high-pass filter to the first motion data.

According to a still further aspect of an exemplary embodiment, a method of processing a magnetic resonance (MR) image includes acquiring projection data with respect to a first cross-section, the first cross-section not showing an aorta or a boundary of an abdomen of an object, the aorta and the boundary of the abdomen overlapping each other, by applying a bipolar gradient magnetic field to the object; acquiring motion data representing a first motion corresponding to a heartbeat of the objection and a second motion corresponding to a respiration of the object based on phase signal data obtained based on the projection data; and acquiring magnetic resonance imaging (MRI) data based on the motion data.

The acquiring of the motion data may include: acquiring the first motion data based on first phase signal data corresponding to the aorta; and acquiring the second motion data based on second phase signal data corresponding to a boundary of an abdomen.

The acquiring of the MRI data may include acquiring the MRI data during a time interval determined based on the first motion data and the second motion data.

The acquiring of the MRI data may include acquiring the MRI data with respect to a second cross-section different from the first cross-section.

The method may further include controlling the bipolar gradient magnetic field to be applied in an aortic direction, the aortic direction being a direction in which the aorta is disposed within the object.

The method may further include displaying at least one of a first image generated based on the motion data and a second image generated based on the MRI data.

BRIEF DESCRIPTION OF THE DRAWINGS

These and/or other aspects will become apparent and more readily appreciated from the following description of the exemplary embodiments, taken in conjunction with the accompanying drawings in which:

FIG. 1 is a block diagram of a general magnetic resonance imaging (MRI) system;
FIG. 2 is a block diagram of an MRI apparatus according to an exemplary embodiment;
FIG. 3 is a block diagram of an MRI apparatus according to an exemplary embodiment;

FIG. 4 is a flowchart of a method of processing a medical image according to an exemplary embodiment;

FIG. 5 is a pulse sequence schematic diagram for obtaining an MR image according to an exemplary embodiment;

FIG. 6 illustrates first and second cross-sections according to an exemplary embodiment;

FIG. 7A illustrates a first cross-section according to an exemplary embodiment;

FIG. 7B illustrates a first cross-section according to an exemplary embodiment;

FIG. 7C illustrates a first cross-section according to an exemplary embodiment;

FIG. 8 is a flowchart of a method of processing an MR image according to an exemplary embodiment;

FIG. 9A illustrates phase signal data according to an exemplary embodiment;

FIG. 9B illustrates a first image generated based on motion data according to an exemplary embodiment;

FIG. 9C illustrates a third motion image according to an exemplary embodiment; and

FIG. 10 illustrates a configuration of a communicator according to an exemplary embodiment.

DETAILED DESCRIPTION

Advantages and features of one or more exemplary embodiments of the present disclosure and methods of accomplishing the same may be understood more readily by reference to the following detailed description of the exemplary embodiments and the accompanying drawings. In this regard, the present exemplary embodiments may have different forms and should not be construed as being limited to the descriptions set forth herein. Rather, these exemplary embodiments are provided so that this disclosure will be thorough and complete and will fully convey the concept of the present exemplary embodiments to one of ordinary skill in the art, and the present disclosure will only be defined by the appended claims.

Terms used herein will now be briefly described and then one or more exemplary embodiments of the present disclosure will be described in detail.

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, some terms may be arbitrarily selected by the applicant, and in this case, the meaning of the selected terms will be described in detail in the detailed description of the disclosure. Thus, the terms used herein have to be defined based on the meaning of the terms together with the description throughout the specification.

When a part "includes" or "comprises" an element, unless there is a particular description contrary thereto, the part can further include other elements, not excluding the other elements. Also, the terms component or "unit" in the exemplary embodiments of the present disclosure may mean a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

Reference will now be made in detail to exemplary embodiments, examples of which are illustrated in the accompanying drawings. In the following description, well-known functions or constructions are not described in detail so as not to obscure the exemplary embodiments with unnecessary detail. Expressions such as "at least one of," when preceding a list of elements, modify the entire list of elements and do not modify the individual elements of the list.

Throughout the specification, an "image" may denote multi-dimensional data composed of discrete image elements (for example, pixels in a two-dimensional image and voxels in a three-dimensional image). For example, the image may be a medical image of an object captured by an X-ray apparatus, a computed tomography (CT) apparatus, a magnetic resonance imaging (MRI) apparatus, an ultrasound diagnosis apparatus, or another medical imaging apparatus.

Furthermore, in the present specification, an "object" may be a human, an animal, or a part of a human or animal. For example, the object may be an organ (e.g., the liver, the heart, the womb, the brain, a breast, or the abdomen), a blood vessel, or a combination thereof. Furthermore, the "object" may be a phantom. The phantom means a material having a density, an effective atomic number, and a volume that are approximately the same as those of an organism. For example, the phantom may be a spherical phantom having properties similar to the human body.

Furthermore, in the present specification, a "user" may be, but is not limited to, a medical expert, such as a medical doctor, a nurse, a medical laboratory technologist, or a technician who repairs a medical apparatus.

Furthermore, in the present specification, an "MR image" may refer to an image of an object obtained by using the nuclear magnetic resonance principle.

Furthermore, in the present specification, a "pulse sequence" refers to continuity of signals repeatedly applied by an MRI apparatus. The pulse sequence may include a time parameter of a radio frequency (RF) pulse, for example, repetition time (TR) or echo time (TE).

Furthermore, in the present specification, a "pulse sequence schematic diagram" shows an order of events that occur in an MRI apparatus. For example, the pulse sequence schematic diagram may be a diagram showing an RF pulse, a gradient magnetic field, a magnetic resonance (MR) signal, or the like according to time.

An MRI system is an apparatus for acquiring a sectional image of a part of an object by expressing, in a contrast comparison, a strength of a MR signal with respect to a radio frequency (RF) signal generated in a magnetic field having a specific strength. For example, if an RF signal that only resonates a specific atomic nucleus (for example, a hydrogen atomic nucleus) is emitted for an instant toward the object placed in a strong magnetic field and then such emission stops, an MR signal is emitted from the specific atomic nucleus, and thus the MRI system may receive the MR signal and acquire an MR image. The MR signal denotes an RF signal emitted from the object. An intensity of the MR signal may be determined according to a density of a predetermined atom (for example, hydrogen) of the object, a relaxation time T1, a relaxation time T2, and a flow of blood or the like.

MRI systems include characteristics different from those of other imaging apparatuses. Unlike imaging apparatuses such as CT apparatuses that acquire images according to a direction of detection hardware, MRI systems may acquire 2D images or 3D volume images that are oriented toward an optional point. MRI systems do not expose objects or examiners to radiation, unlike CT apparatuses, X-ray apparatuses, position emission tomography (PET) apparatuses, and single photon emission CT (SPECT) apparatuses, may acquire images having high soft tissue contrast, and may acquire neurological images, intravascular images, musculoskeletal images, and oncologic images that are required to precisely capturing abnormal tissues.

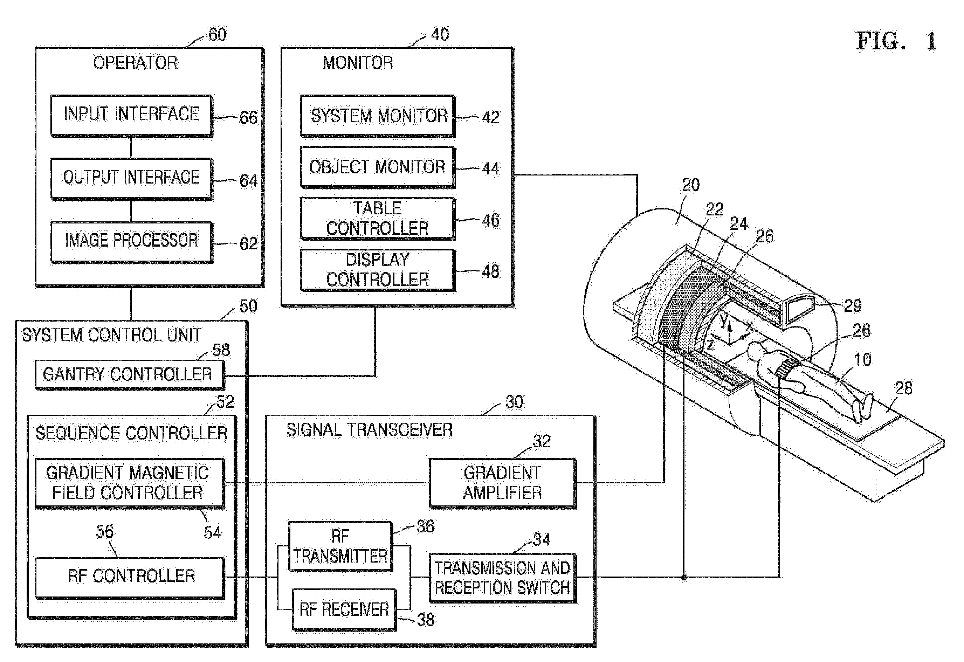

FIG. 1 is a block diagram of a general MRI system. Referring to FIG. 1, the general MRI system may include a gantry 20, a signal transceiver 30, a monitor 40, a system controller 50, and an operator 60.

The gantry 20 prevents external emission of electromagnetic waves generated by a main magnet 22, a gradient coil 24, and an RF coil 26. A magnetostatic field and a gradient magnetic field are formed in a bore in the gantry 20, and an RF signal is emitted toward an object 10.

The main magnet 22, the gradient coil 24, and the RF coil 26 may be arranged in a predetermined direction of the gantry 20. The predetermined direction may be a coaxial cylinder direction. The object 10 may be disposed on a table 28 that is capable of being inserted into a cylinder along a horizontal axis of the cylinder.

The main magnet 22 generates a magnetostatic field or a static magnetic field for aligning magnetic dipole moments of atomic nuclei of the object 10 in a constant direction. A precise and accurate MR image of the object 10 may be obtained due to a magnetic field generated by the main magnet 22 being strong and uniform.

The gradient coil 24 includes X, Y, and Z coils for generating gradient magnetic fields in X-, Y-, and Z-axis directions crossing each other at right angles. The gradient coil 24 may provide location information of each region of the object 10 by differently inducing resonance frequencies according to the regions of the object 10.

The RF coil 26 may emit an RF signal toward a patient and receive an MR signal emitted from the patient. In detail, the RF coil 26 may transmit, toward atomic nuclei included in the patient and having precessional motion, an RF signal having the same frequency as that of the precessional motion, stop transmitting the RF signal, and then receive an MR signal emitted from the atomic nuclei included in the patient.

For example, in order to transit an atomic nucleus from a low energy state to a high energy state, the RF coil 26 may generate and apply an electromagnetic wave signal that is an RF signal corresponding to a type of the atomic nucleus, to the object 10. When the electromagnetic wave signal generated by the RF coil 26 is applied to the atomic nucleus, the atomic nucleus may transit from the low energy state to the high energy state. Then, when electromagnetic waves generated by the RF coil 26 disappear, the atomic nucleus to which the electromagnetic waves were applied transits from the high energy state to the low energy state, thereby emitting electromagnetic waves having a Lamor frequency. In other words, when the applying of the electromagnetic wave signal to the atomic nucleus is stopped, an energy level of the atomic nucleus is changed from a high energy level to a low energy level, and thus the atomic nucleus may emit electromagnetic waves having a Lamor frequency. The RF coil 26 may receive electromagnetic wave signals from atomic nuclei included in the object 10.

In some exemplary embodiments, the RF coil 26 may be one RF transmitting and receiving coil having both a function of generating electromagnetic waves each having an RF that corresponds to a type of an atomic nucleus and a function of receiving electromagnetic waves emitted from an atomic nucleus. In some exemplary embodiments, the RF coil 26 may be a transmission RF coil having a function of generating electromagnetic waves each having an RF that corresponds to a type of an atomic nucleus, and a reception RF coil having a function of receiving electromagnetic waves emitted from an atomic nucleus.

The RF coil 26 may be fixed to the gantry 20 or may be detachable. When the RF coil 26 is detachable, the RF coil 26 may be an RF coil for a part of the object, such as a head RF coil, a chest RF coil, a leg RF coil, a neck RF coil, a shoulder RF coil, a wrist RF coil, or an ankle RF coil.

The RF coil 26 may communicate with an external apparatus via wires and/or wirelessly, and may also perform dual tune communication according to a communication frequency band.

The RF coil 26 may be a birdcage coil, a surface coil, or a transverse electromagnetic (TEM) coil according to structures.

The RF coil 26 may be a transmission exclusive coil, a reception exclusive coil, or a transmission and reception coil according to methods of transmitting and receiving an RF signal.

The RF coil 26 may be an RF coil having various numbers of channels, such as 16 channels, 32 channels, 72 channels, and 144 channels.

The gantry 20 may further include a display 29 disposed outside the gantry 20 and a display disposed inside the gantry 20. The gantry 20 may provide predetermined information to the user or the object 10 through the display 29 and the display respectively disposed outside and inside the gantry 20.

The signal transceiver 30 may control the gradient magnetic field formed inside the gantry 20, i.e., in the bore, according to a predetermined MR sequence, and control transmission and reception of an RF signal and an MR signal.

The signal transceiver 30 may include a gradient amplifier 32, a transmission and reception switch 34, an RF transmitter 36, and an RF receiver 38.

The gradient amplifier 32 drives the gradient coil 24 included in the gantry 20, and may supply a pulse signal for generating a gradient magnetic field to the gradient coil 24 under the control of a gradient magnetic field controller 54. By controlling the pulse signal supplied from the gradient amplifier 32 to the gradient coil 24, gradient magnetic fields in X-, Y-, and Z-axis directions may be synthesized.

The RF transmitter 36 and the RF receiver 38 may drive the RF coil 26. The RF transmitter 36 may supply an RF pulse in a Lamor frequency to the RF coil 26, and the RF receiver 38 may receive an MR signal received by the RF coil 26.

The transmission and reception switch 34 may adjust transmitting and receiving directions of the RF signal and the MR signal. For example, the transmission and reception switch 34 may emit the RF signal toward the object 10 through the RF coil 26 during a transmission mode, and receive the MR signal from the object 10 through the RF coil 26 during a reception mode. The transmission and reception switch 34 may be controlled by a control signal output by an RF controller 56.

The monitor 40 may monitor or control the gantry 20 or devices mounted on the gantry 20. The monitor 40 may include a system monitor 42, an object monitor 44, a table controller 46, and a display controller 48.

The system monitor 42 may monitor and control a state of the magnetostatic field, a state of the gradient magnetic field, a state of the RF signal, a state of the RF coil 26, a state of the table 28, a state of a device measuring body information of the object 10, a power supply state, a state of a thermal exchanger, and a state of a compressor.

The object monitor 44 monitors a state of the object 10. In detail, the object monitor 44 may include a camera for observing a movement or position of the object 10, a respiration measurer for measuring the respiration of the object 10, an electrocardiogram (ECG) measurer for measuring the electrical activity of the object 10, or a temperature measurer for measuring a temperature of the object 10.

The table controller 46 controls a movement of the table 28 where the object 10 is positioned. The table controller 46 may control the movement of the table 28 according to a sequence control of a sequence controller 50. For example, during moving imaging of the object 10, the table controller 46 may continuously or discontinuously move the table 28 according to the sequence control of the sequence controller 50, and thus the object 10 may be photographed in a field of view (FOV) larger than that of the gantry 20.

The display controller 48 controls the display 29 disposed outside the gantry 20 and the display disposed inside the gantry 20. In detail, the display controller 48 may control the display 29 and the display to be on or off, and may control a screen image to be output on the display 29 and the display. Also, when a speaker is located inside or outside the gantry 20, the display controller 48 may control the speaker to be on or off, or may control sound to be output via the speaker.

The system controller 50 may include the sequence controller 52 for controlling a sequence of signals formed in the gantry 20, and a gantry controller 58 for controlling the gantry 20 and the devices mounted on the gantry 20.

The sequence controller 52 may include the gradient magnetic field controller 54 for controlling the gradient amplifier 32, and the RF controller 56 for controlling the RF transmitter 36, the RF receiver 38, and the transmission and reception switch 34. The sequence controller 52 may control the gradient amplifier 32, the RF transmitter 36, the RF receiver 38, and the transmission and reception switch 34 according to a pulse sequence received from the operator 60. Here, the pulse sequence includes information used to control the gradient amplifier 32, the RF transmitter 36, the RF receiver 38, and the transmission and reception switch 34. For example, the pulse sequence may include information about a strength, an application time, and application timing of a pulse signal applied to the gradient coil 24.

The operator 60 may request the system controller 50 to transmit pulse sequence information while controlling an overall operation of the MRI system.

The operator 60 may include an image processor 62 for receiving and processing the MR signal received by the RF receiver 38, an output interface 64, and an input interface 66.

The image processor 62 may process the MR signal received from the RF receiver 38 so as to generate MR image data of the object 10.

The image processor 62 receives the MR signal received by the RF receiver 38 and performs any one of various signal processes, such as amplification, frequency transformation, phase detection, low frequency amplification, and filtering, on the received MR signal.

The image processor 62 may arrange digital data in a k space (for example, also referred to as a Fourier space or a frequency space) of a memory, and rearrange the digital data into image data via 2D or 3D Fourier transformation.

If needed, the image processor 62 may perform a composition process or difference calculation process on the image data. The composition process may include an addition process on a pixel or a maximum intensity projection (MIP) process. The image processor 62 may store not only the rearranged image data but also image data on which a composition process or a difference calculation process is performed, in a memory or an external server.

The image processor 62 may perform any of the signal processes on the MR signal in parallel. For example, the image processor 62 may perform a signal process on a plurality of MR signals received by a multichannel RF coil in parallel so as to rearrange the plurality of MR signals into image data.

The output interface 64 may output image data generated or rearranged by the image processor 62 to the user. The output interface 64 may also output information required for the user to manipulate the MRI system, such as a user interface (UI), user information, or object information. The output interface 64 may be a speaker, a printer, a cathode-ray tube (CRT) display, a liquid crystal display (LCD), a plasma display panel (PDP), an organic light-emitting device (OLED) display, a field emission display (FED), a light-emitting diode (LED) display, a vacuum fluorescent display (VFD), a digital light processing (DLP) display, a flat panel display (FPD), a 3-dimensional (3D) display, a transparent display, or any one of other various output devices that are well known to one of ordinary skill in the art.

The user may input object information, parameter information, a scan condition, a pulse sequence, or information about image composition or difference calculation by using the input interface 66. The input interface 66 may be a keyboard, a mouse, a track ball, a voice recognizer, a gesture recognizer, a touch screen, or any one of other various input devices that are well known to one of ordinary skill in the art.

The signal transceiver 30, the monitor 40, the system controller 50, and the operator 60 are separate components in FIG. 1, but it will be obvious to one of ordinary skill in the art that respective functions of the signal transceiver 30, the monitor 40, the system controller 50, and the operator 60 may be performed by another component. For example, the image processor 62 converts the MR signal received from the RF receiver 38 into a digital signal in FIG. 1, however, in some exemplary embodiments, the conversion of the MR signal into the digital signal may be performed by the RF receiver 38 or the RF coil 26.

The gantry 20, the RF coil 26, the signal transceiver 30, the monitor 40, the system controller 50, and the operator 60 may be connected to each other by wire or wirelessly, and when they are connected wirelessly, the MRI system may further include an apparatus for synchronizing clock signals therebetween. Communication between the gantry 20, the RF coil 26, the signal transceiver 30, the monitor 40, the system controller 50, and the operator 60 may be performed by using a high-speed digital interface, such as low voltage differential signaling (LVDS), asynchronous serial communication, such as a universal asynchronous receiver transmitter (UART), a low-delay network protocol, such as error synchronous serial communication or a controller area network (CAN), or optical communication.

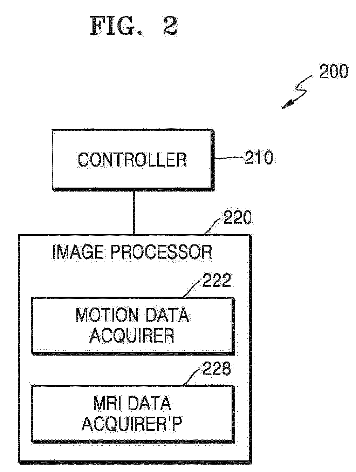

FIG. 2 is a block diagram of an MRI apparatus 200 according to an exemplary embodiment.

The MRI apparatus 200 according to the present exemplary embodiment may be equipment that captures medical images of an object based on the principle of nuclear magnetic resonance (NMR) and processes the captured medical images. A user, e.g., a medical practitioner may diagnose a patient's health conditions and disease by using a medical image output from the MRI apparatus 200.

Referring to FIG. 2, the MRI apparatus 200 may include a controller 210 and an image processor 220.

According to an exemplary embodiment, the controller 210 may control a bipolar gradient magnetic field to be applied to an object. Furthermore, the controller 210 may control a bipolar gradient magnetic field to be applied in the same direction that an aorta included in the object runs, which may be referred to as an aortic direction. When the bipolar gradient magnetic field is applied to the aorta, spins in blood flow within the aorta may undergo a phase change.

The following Equation (1) represents a relation between a phase of a moving spin included in an object and a magnitude G of a gradient magnetic field:

$$\phi = -\gamma \int_{t} G(\tau)X(\tau)d\tau \qquad \dots (1)$$

where the phase of a moving spin is the phase of a spin of an atomic nucleus that emits an MR signal, G represents the magnitude of a gradient magnetic field with respect to time, X represents a position of the spin, and $\gamma$ represents a gyromagnetic ratio.

When an object moves due to a heartbeat and respiration, positions of spins in the object may change. According to Equation (1), a change in positions of spins may be measured by measuring a change in phase of the spins. In other words, motions of the object due to heartbeat and respiration may be measured by measuring a change in phase of spins in the object.

In detail, a heartbeat may greatly affect blood flow within an aorta. By measuring a change in phase of spins corresponding to blood flow in an aorta, motion caused by the heartbeat may be measured. According to an exemplary embodiment, the user may measure a change in phase of spins corresponding to blood flow in a part of the aorta that is located close to the heart from among parts of the aorta.

Furthermore, respiration may significantly affect motion of the abdomen or chest. According to an exemplary embodiment, the user may measure motion due to respiration by measuring a change in phase of spins corresponding to the abdomen or chest.

In addition, a magnitude of a bipolar gradient magnetic field that is applied to the object may be determined by a velocity encoding (VENC). A VENC that is measured in cm/sec may represent a maximum measurable velocity of motion. According to an exemplary embodiment, the VENC may be determined by a blood flow velocity in the aorta. The following Equation (2) represents a relation between a VENC and intensity G of a gradient magnetic field:

$$VENC = \pi/2\gamma \int_t G(\tau)\tau d\tau \qquad \dots (2)$$

where G is the intensity of a gradient magnetic field with respect to time and $\gamma$ is a gyromagnetic ratio.

The controller 210 may control a bipolar gradient magnetic field whose intensity is determined by Equation (2) above to be applied in the same direction that the aorta of an object runs. The controller 210 described with reference to FIG. 2 may include a gradient magnetic field controller 54 described with reference to FIG. 1.

According to an exemplary embodiment, the image processor 220 may acquire motion data representing motions due to heartbeat and respiration of an object based on phase signal data acquired by applying a bipolar gradient magnetic field to the object and obtain MRI data based on the motion data.

In detail, referring to FIG. 2, the image processor 220 may include a motion data acquirer 222 and an MRI data acquirer 228.

According to an exemplary embodiment, the motion data acquirer 222 may acquire motion data representing motions due to a heartbeat and respiration of an object based on phase signal data acquired by applying a bipolar gradient magnetic field to the object.

According to an exemplary embodiment, the phase signal data may include data representing a change in phase of spins included in the object with respect to time.

For example, the phase signal data may include first phase signal data corresponding to an aorta and second phase signal data corresponding to a boundary of the abdomen. In detail, the first phase signal data may include data representing a change in phase of spins corresponding to the aorta. Furthermore, the second phase signal data may include data representing a change in phase of spins corresponding to a boundary of the abdomen. As another example, the phase signal data may include data representing a change in phase of spins corresponding to a boundary of the chest.

According to an exemplary embodiment, the motion data may include data representing motion caused by heartbeat of the object and data representing motion caused by respiration thereof.

For example, the motion data acquirer 222 may acquire first motion data representing motion caused by heartbeat of the object based on first phase signal data corresponding to the aorta. Furthermore, the motion data acquirer 222 may acquire second motion data representing motion caused by respiration of the object based on second phase signal data corresponding to a boundary of the abdomen. In detail, the first motion data may include data presenting a change in blood flow within the aorta. Furthermore, the second motion data may include data representing motion of the boundary of the abdomen.

As another example, the motion data may include data representing motion of a boundary of the chest. In other words, the motion data acquirer 222 may acquire motion data representing motion of the boundary of the chest of the object based on data representing a change in phase of spins corresponding to the boundary of the chest.

In addition, the motion data acquirer 222 may acquire the first motion data simultaneously with the second motion data based on phase signal data.

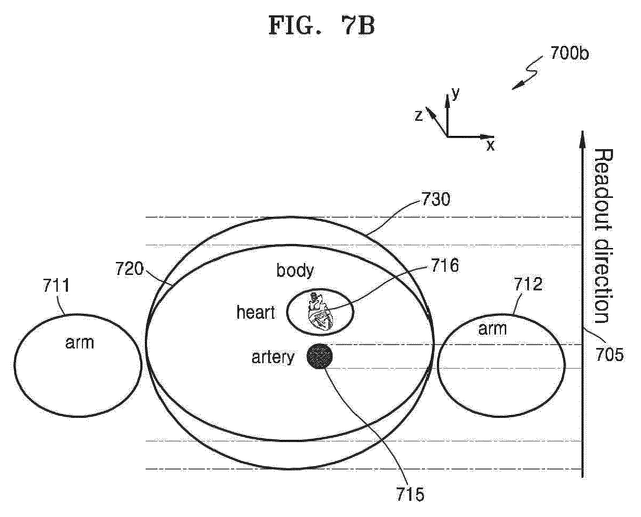

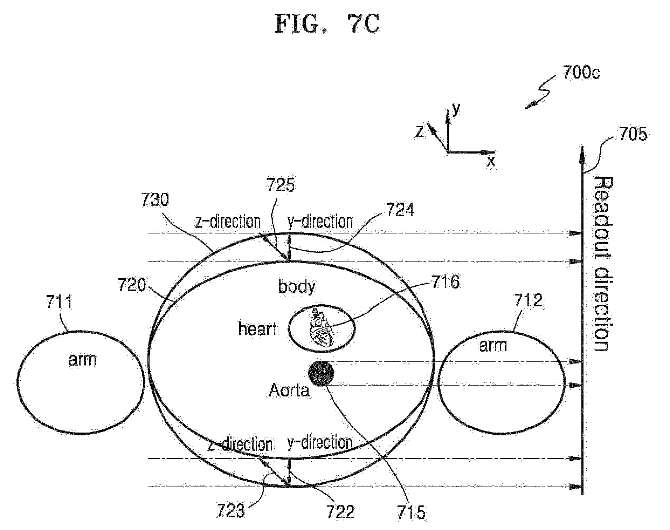

According to another exemplary embodiment, the motion data acquirer 222 may acquire projection data with respect to a first cross-section by applying a bipolar gradient magnetic field to an object. In some exemplary embodiments, projection data with respect to the first cross-section may not show the aorta and a boundary of a torso of the object, if for example they overlap each other. In this case, the first cross-section may be a plane including a cross-section of the aorta and the boundary of the torso of the object. Furthermore, the boundary of the torso in the first cross-section may include boundaries of the abdomen and chest. For example, the first cross-section may be a first cross-section (e. g., 700c of FIG. 7C). When projection data with respect to the first cross-section 700c is acquired along a second readout direction 705 as shown in FIG. 7B, the projection data with respect to the first cross-section 700c may not show the aorta and the boundary of the torso in the object, as they overlap each other.

The motion data acquirer 222 may acquire motion data representing motions due to a heartbeat and respiration of the object based on phase signal data that is obtained based on projection data with respect to a first cross-section.

In detail, the motion data acquirer 222 may acquire k-space data with respect to a first cross-section (e.g., 700c of FIG. 7C). A DC line may be acquired based on the acquired k-space data. The DC line contains values of k-space data with respect to a change in a y-value when an x-value in a k-space is zero (0). Projection data with respect to a Y-axis in the first cross-section 700c may be acquired based on the DC line. The projection data with respect to the Y-axis in the first cross-section 700c may be acquired by performing an inverse Fourier transform on the DC line. The projection data may be phase signal data of spins located in the first cross-section 700c, which is acquired during a specific time interval.

According to an exemplary embodiment, the MRI data acquirer 228 may acquire MRI data based on motion data representing motions caused by heartbeat and respiration of an object.

In this case, the MRI data may include an MR signal for generating an MR image of the object. Furthermore, the MRI data may include other types of data acquired based on the MR signal. For example, the MRI data may include data necessary to generate an image of a specific cross-section based on the MR signal.

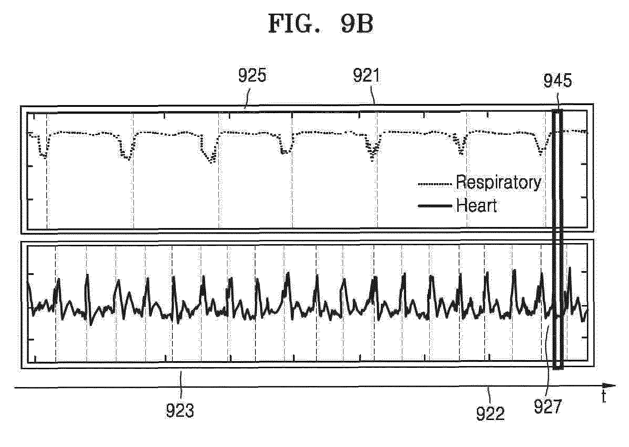

For example, the MRI data acquirer 228 may acquire MRI data during a time interval determined based on first motion data representing motion due to heartbeat of the object and second motion data representing motion due to respiration of the object. A time interval (e.g., 945 of FIG. 9B) determined by first motion data representing motion due to heartbeat of an object and second motion data representing motion due to respiration of the object will be described in detail below with reference to FIG. 9B.

The user may obtain an MR image that suffers minimized degradation in quality due to reduced motion of the object by acquiring MRI data during a time interval (e.g., 945 of FIG. 9B).

The image processor 220 may include an image processor 62 described with reference to FIG. 1.

The MRI apparatus 200 may acquire motion data representing motions due to heartbeat and respiration of the object based on an MR signal obtained by performing an MRI scan on the object. Furthermore, the MRI apparatus 200 may measure a heartbeat and respiration of the object without needing to attach a gating device for measuring the heartbeat and respiration thereof while simultaneously obtaining an MR image at a time point when motion of the object is minimized.

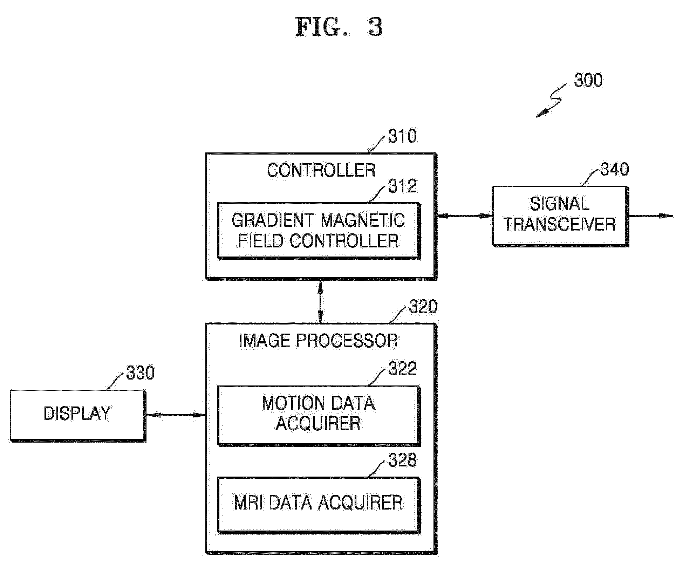

FIG. 3 is a block diagram of an MRI apparatus 300 according to another exemplary embodiment.

Referring to FIG. 3, the MRI apparatus 300 may include a controller 310, an image processor 320, a display 330, and a signal transceiver 340. The controller 310 may include a gradient magnetic field controller 312.

The gradient magnetic field controller 312 may include a gradient magnetic field controller 54 described with reference to FIG. 1. The image processor 320 may include a motion data acquirer 322 and an MRI image data acquirer 328.

The controller 310 and the image processor 320 of the MRI apparatus 300 may respectively correspond to the controller 210 and the image processor 220 described with reference to FIG. 2. Thus, descriptions already provided with respect to FIG. 2 will be omitted below.

The MRI apparatus 300 may further include the display 300 and the signal transceiver 340.

The display 330 may display at least one of a first image generated based on motion data and a second image generated based on MRI data. The display 330 may include the output interface 64 described with reference to FIG. 1.

The signal transceiver 340 may transmit a signal for applying a gradient magnetic field based on a signal from the controller 310. In other words, the signal transceiver 340 may transmit a signal for applying a gradient magnetic field having an X-, Y-, or Z-axis direction to inside a gantry in which the object is placed. For example, the signal transceiver 340 may control generation of a pulse signal for applying a gradient magnetic field. Furthermore, the signal transceiver 340 may control a pulse signal to be supplied to a coil and control an RF signal emitted from an atomic nucleus included in the object to be received. The RF signal may be an MR signal emitted by the object.

The signal transceiver 340 may include the signal transceiver 30 described with reference to FIG. 1.

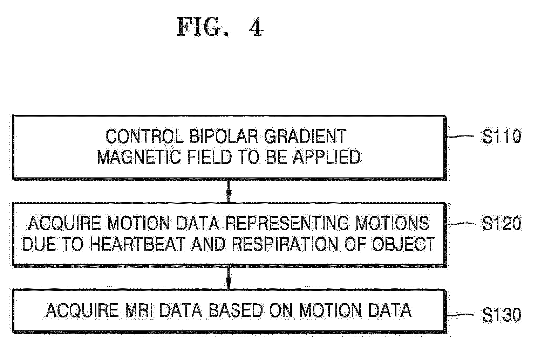

FIG. 4 is a flowchart of a method of processing a medical image according to an exemplary embodiment.

Referring to FIG. 4, the MRI apparatus 200 (or 300) may control a bipolar gradient magnetic field to be applied to an object (S110).

According to an exemplary embodiment, in operation S110, the MRI apparatus 200 (or 300) may control a bipolar gradient magnetic field to be applied to an object in the same direction that an aorta of the object runs.

The object may move due to heartbeat and respiration. In detail, blood flow within an aorta may change due to the heartbeat, and a torso of the object may move due to respiration.

In addition, when the object moves, positions of spins included in the object may change. A change in positions of spins may be measured by measuring a change in phase of the spins. In other words, motions due to a heartbeat and respiration may be measured based on a change in phase of spins included in the object.

The MRI apparatus 200 (300) may acquire motion data representing motions due to heartbeat and respiration of the object based on phase signal data obtained by applying a bipolar gradient magnetic field to the object (S120).

According to an exemplary embodiment, the phase signal data may include data representing a change in phase of spins representing a change in phase of spins included in the object with respect to time. For example, the phase signal data may include first phase signal data corresponding to an aorta and second phase signal data corresponding to a boundary of the abdomen.

The motion data may include data representing motion caused by heartbeat of the object and data representing motion caused by respiration thereof.

For example, in operation S120, the MRI apparatus 200 (or 300) may acquire first motion data representing motion caused by heartbeat of the object based on first phase signal data corresponding to the aorta.

Furthermore, in operation S120, the MRI apparatus 200 (or 300) may acquire second motion data representing motion caused by respiration of the object based on second phase signal data corresponding to a boundary of the abdomen. More specifically, the first motion data may include data presenting a change in blood flow within the aorta. Furthermore, the second motion data may include data representing motion of the boundary of the abdomen.

In addition, in operation S120, the MRI apparatus 200 (or 300) may acquire projection data with respect to a first cross-section in which the aorta and a boundary of the abdomen of the object overlap each other and thus are not shown, by applying a bipolar gradient magnetic field to the object.

The MRI apparatus 200 (300) may acquire MRI data based on the motion data (S130).

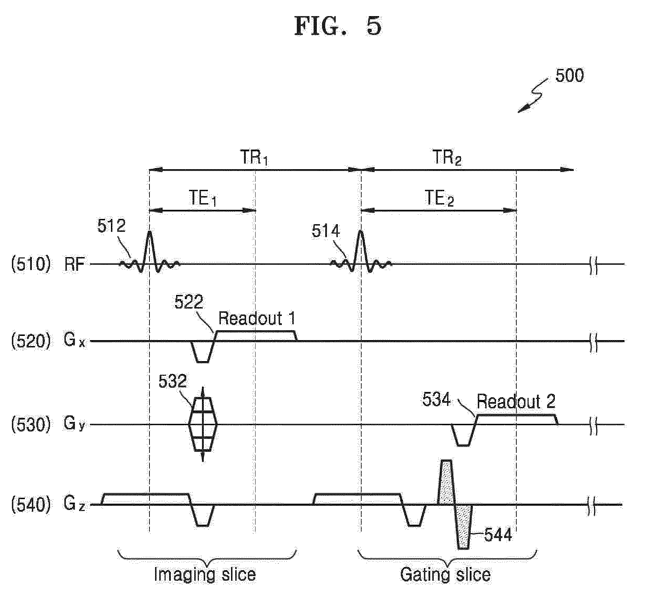

FIG. 5 is a pulse sequence schematic diagram 500 for obtaining an MR image according to an exemplary embodiment.

In detail, FIG. 5 illustrates the pulse sequence diagram 500 for acquiring motion data and MRI data via the MRI apparatus 200 according to an exemplary embodiment. In the pulse sequence diagram 500, the abscissa and ordinate respectively denote time and intensity of an RF signal or magnetic field.

The MRI apparatus 200 (or 300) may apply an RF pulse signal 510 to the RF coil (26 of FIG. 1). The RF pulse 512 may be an $\alpha°$ pulse having a frequency of $\omega 0$, and an RF pulse 514 may be an $\alpha°$ pulse having a frequency of $\omega 0 + \Delta\omega$.

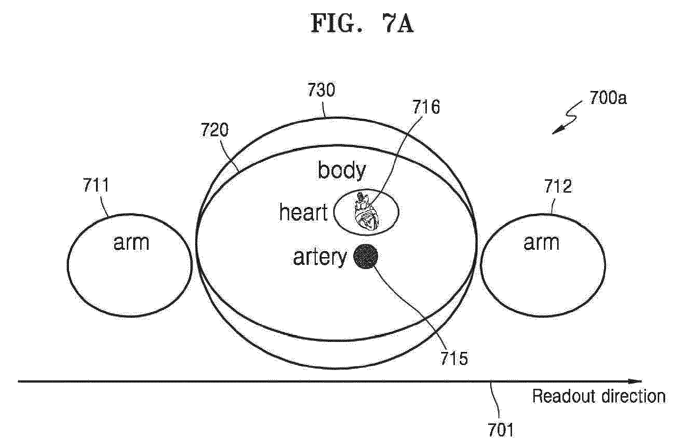

The MRI apparatus 200 may apply a gradient $G_x$ 520 to create a gradient magnetic field along the X-axis. Referring to FIG. 5, the MRI apparatus 200 may apply a first readout gradient 522 along the X-axis. For example, the first readout gradient 522 may be applied in a first readout direction (e.g., 701 of FIG. 7A). The MRI apparatus 200 may acquire MRI data with respect to the object by using the first readout gradient 522 applied along the first readout direction 701.

The MRI apparatus 200 may apply a gradient Gy 530 to create a gradient magnetic field along the Y-axis. Referring to FIG. 5, the MRI apparatus 200 may apply a phase encoding gradient 532 for phase encoding along the Y-axis. Furthermore, the MRI apparatus 200 may apply a second readout gradient 534 along the Y-axis. For example, the second readout gradient 534 may be applied in a second readout direction (e.g., 705 of FIG. 7B). The MRI apparatus 200 may acquire motion data with respect to the object by using the second readout gradient 534 applied along the second readout direction 705.

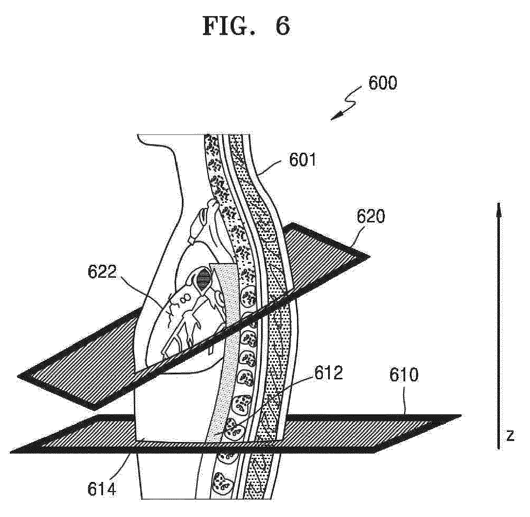

The MRI apparatus 200 may apply a gradient $G_z$ 540 to create a gradient magnetic field along the Z-axis. The MRI apparatus 200 may apply the gradient $G_z$ 540 to select an imaging slice. The imaging slice may include a cross-section forming a first angle with the Z-axis. For example, the first angle may include an angle formed between the Z-axis and a long axis of the heart. The cross-section forming the first angle with the Z-axis may be a second cross-section 620 shown in FIG. 6.

Furthermore, the MRI apparatus 200 may apply a gradient to select a gating slice. The gating slice may include a first cross-section in which the aorta and a boundary of the abdomen of the object overlap each other and thus are not shown. The first cross-section may be a first cross-section 610 shown in FIG. 6.

In addition, the MRI apparatus 200 may apply a bipolar gradient magnetic field 544 along the Z-axis direction. The Z-axis direction may correspond to a direction in which the aorta runs. When the bipolar gradient magnetic field 544 is applied in the direction in which the aorta runs, phase of spins corresponding the aorta may change.

A magnitude of the bipolar gradient magnetic field applied in the Z-axis direction may be determined by a VENC. A VENC (cm/sec) may represent a maximum measurable velocity of motion. According to an exemplary embodiment, the VENC may be determined by a blood flow velocity in the aorta.

FIG. 6 illustrates the first and second cross-sections 610 and 620 of an object 600 according to an exemplary embodiment.

Referring to FIG. 6, the first cross-section may be perpendicular to the Z-axis. The first cross-section 610 may include an aorta 612 of the object 600 and a boundary 614 of a torso of the object 600. In this case, the boundary 614 of the torso of the object 600 may include boundaries of the abdomen and chest of the object 600.

The abdomen is surrounded by abdominal muscles and includes some of digestive system organs and urinary system organs. The chest may include respiratory system organs, esophagus, thymus, and thoracic muscles. The diaphragm may lie between the abdomen and the chest. The boundary 614 of the torso of the object 600 may move as lungs of the object 600 expand and contract. In this case, after acquiring phase signal data with respect to the first cross-section 610, motion data may be acquired based on the phase signal data.

The second cross-section 620 may form a first angle with the Z-axis and include a heart 622 of the object 600. As shown in FIG. 6, the first angle may be an angle formed between the Z-axis and a long axis of the heart 622.

FIG. 7A illustrates a first cross-section 700a according to an exemplary embodiment.

As described with reference to FIG. 6, the first cross-section 700a may be a cross-section perpendicular to the Z-axis.

The first cross-section 700a may show arms 711 and 712, a contracted torso 720, and an expanded torso 730 of an object. The contracted or expanded torso 720 or 730 may include the abdomen and the chest of the object. In other words, a line formed where the first cross-section 700a and the boundary of the contracted or expanded torso 720 or 730 meet each other may include boundaries of the abdomen and chest. Furthermore, an aorta 715 may be depicted in the first cross-section 700a. According to an exemplary embodiment, a heart 716 may be shown in the first cross-section 700a.

The first readout direction 701 shown in FIG. 7A may correspond to a direction of the gradient magnetic field created by applying the first readout gradient 522 described with reference to FIG. 5. In other words, the first readout direction 701 may correspond to the X-axis direction. Furthermore, the first readout direction 701 may be a readout direction for acquiring MRI data with respect to the object.

FIG. 7B illustrates a first cross-section 700b according to an exemplary embodiment.

As described above with reference to FIG. 7A, the first cross-section 700b may show arms 711 and 712, a contracted torso 720, and an expanded torso 730 of an object. The torso may include the chest and abdomen of the object. Furthermore, an aorta 715 may be depicted in the first cross-section 700b. According to an exemplary embodiment, a heart 716 may be shown in the first cross-section 700b.

The second readout direction 705 shown in FIG. 7B may correspond to a direction of the gradient magnetic field created by applying the second readout gradient 524 described with reference to FIG. 5. In other words, the second readout direction 705 may correspond to the Y-axis direction. Furthermore, the second readout direction 705 may be a readout direction for acquiring motion data with respect to the object.

According to an exemplary embodiment, the user may obtain an MR image of the object in the first readout direction 701 shown in FIG. 7A and acquire motion data with respect to the object in the second readout direction 705 shown in FIG. 7B. In other words, the first readout direction 701 for obtaining an MR image may be different from the second readout direction 705 for acquiring motion data.

FIG. 7C illustrates the first cross-section 700c according to an exemplary embodiment.

In detail, FIG. 7C shows motion due to respiration of an object.

According to an exemplary embodiment, an MRI apparatus may acquire k-space data with respect to the first cross-section 700c in a second readout direction 705. A DC line may be acquired based on the acquired k-space data. The DC line contains values of k-space data with respect to a change in a y-value when an x-value in a k-space is zero (0). Projection data with respect to a Y-axis in the first cross-section 700c may be acquired based on the DC line. The projection data with respect to the Y-axis in the first cross-section 700c may be acquired by performing an inverse Fourier transform on the acquired DC line.

The projection data may be phase signal data of spins located in the first cross-section 700c, which is acquired during a specific time interval. The specific time interval may include a repetition time (TR). For example, the TR may be a TR2 shown in FIG. 5. The user may set a TR to 4.7 msec. Furthermore, the specific time interval may include a time to echo (TE). For example, the TE may be a TE2. The user may set a TE to, for example, 2.4msec.

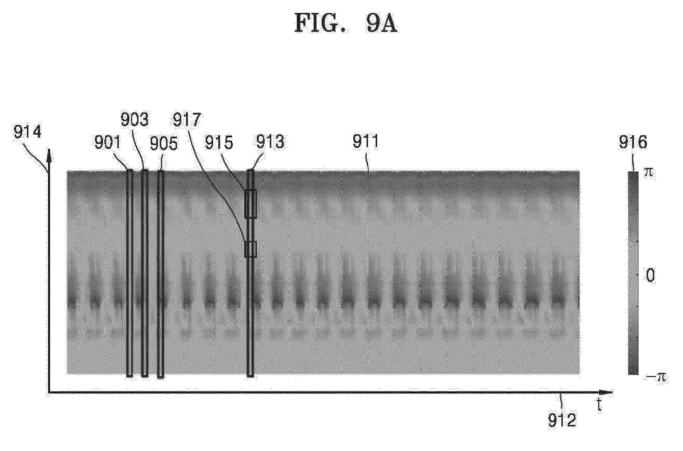

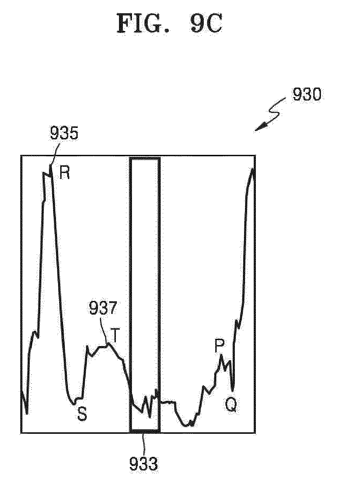

The user may acquire phase signal data based on acquired pieces of projection data. For example, the phase signal data may be acquired based on the projection data with respect to the Y-axis in the first cross-section 700c. For example, the phase signal data (e. g., 911 of FIG. 9) may include a set of the projection data.

The phase signal data may include first phase signal data corresponding to an aorta 715. The first phase signal data may represent a change in phase of spins corresponding to blood flow in the aorta 715 to which a bipolar gradient magnetic field is applied. Furthermore, the first phase signal data may represent motion of blood flow in the aorta 715.

The phase signal data may include second phase signal data corresponding to boundaries of torsos 720 and 730 of the object. In detail, the second phase signal data may include data corresponding to a boundary of the abdomen. Furthermore, the second phase signal data may include data corresponding to a boundary of the chest. The second phase signal data may include data representing a change in phase correlation at a region corresponding to boundaries of the abdomen/chest in the projection data with respect to the Y-axis direction.

Referring to FIG. 7C, the time required to change from the torso 720 contracted due to respiration of the object to the torso 730 expanded due to the respiration and back again may be referred to as one respiratory cycle. When the object breathes, front sides of the torsos 720 and 730 may move in first and second directions 724 and 725 while backsides thereof may move in third and fourth directions 722 and 723. The first and third directions 724 and 722 may correspond to the Y-axis direction, and the second and fourth directions 725 and 723 may correspond to the Z-axis direction. The first cross-section 700c may show motion of a boundary of the abdomen corresponding to the front sides of the torsos 720 and 730 as well as motion of a boundary of the abdomen corresponding to the back sides thereof.

Furthermore, the first cross-section 700c may show motion of a boundary of the chest corresponding to the front sides of the torsos 720 and 730 as well as motion of a boundary of the chest corresponding to the back sides thereof.

In addition, when the first cross-section 700c is perpendicular to the Z-axis, the first cross-section 700c may indicate motion of the boundary of the abdomen corresponding to the front sides of the torsos 720 and 730 as an arrow representing the first direction 724. Furthermore, when the first cross-section 700c is perpendicular to the Z-axis, the first cross-section 700c may indicate motion of the boundary of the abdomen corresponding to the back sides of the torsos 720 and 730 as an arrow representing the third direction 722.

According to an exemplary embodiment, when phase signal data with respect to the Y-axis in the first cross-section 700c is acquired, the first phase signal data corresponding to the aorta and the second phase signal data corresponding to motion of changing boundaries of the abdomen/chest may be acquired so as not to overlap each other.

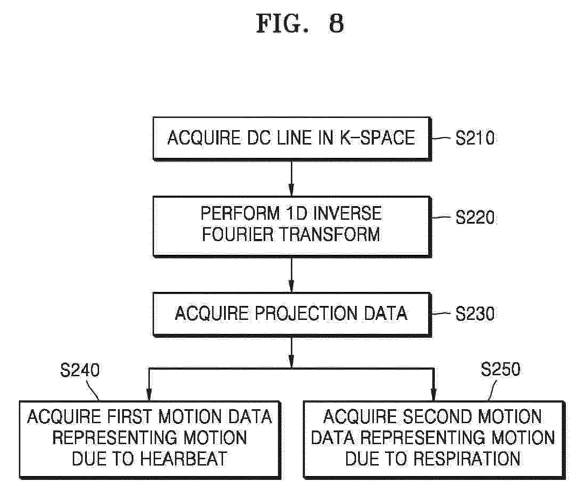

FIG. 8 is a flowchart of a method of processing an MR image according to an exemplary embodiment. In detail, FIG. 8 is a detailed flowchart of operation S120 of acquiring motion data due to heartbeat and respiration of an object described with reference to FIG. 4.

The MRI apparatus 200 (300) may acquire a DC line in a k-space for a first cross-section (S210).

In detail, the MRI apparatus 200 (or 300) may acquire k-space data with respect to the first cross-section (700c of FIG. 7C) along the second readout direction (705 of FIG. 7C). The second readout direction 705 may be different from the first readout direction (701 of FIG. 7A) of an imaging slice for obtaining an MR image. The DC line may be acquired by using values of k-space data with respect to a change in a y-value when an x-value in the k-space is zero.

The MRI apparatus 200 (or 300) may perform a one-dimensional (1D) inverse Fourier transform on the DC line (S220).

By using the 1D inverse Fourier transform, k-space data may be transformed into data in a 1D space.

The MRI apparatus 200 (or 300) may acquire projection data by performing the 1D inverse Fourier transform (S230).

The projection data may be phase signal data of spins located in the first cross-section, which is acquired during a specific time interval.

The MRI apparatus 200 (or 300) may acquire first motion data representing motion due to heartbeat of the object, based on phase signal data that is obtained based on the projection data (S240).

The MRI apparatus 200 (or 300) may acquire second motion data representing motion due to respiration of the object, based on phase signal data that is obtained based on the projection data (S250).

Operation S240 may be performed at the same time as, before, or after operation S250.

FIG. 9A illustrates phase signal data 911 according to an exemplary embodiment.

FIG. 9A shows the phase signal data 911 distributed along a time axis 912 and a first axis 914. As shown in FIG. 9A, phase values of the phase signal data 911 may be indicated using colors based on color coordinates 916.

In detail, the first axis 914 may be an axis corresponding to a readout direction for a first cross-section. The phase signal data 911 may be a set of a plurality of pieces of projection data. For example, the plurality of pieces of projection data may include pieces of projection data 901, 903, 905, and 913. The pieces of projection data 901, 903, 905, and 913 may be 1D data represented by lines. For convenience, referring to FIG. 9A, phase values of the pieces of projection data 901, 903, 905, and 913 may be indicated using colors included within a quadrangular area.

For example, first phase signal data corresponding to an aorta may be acquired based on first partial data 917 in projection data 913. Furthermore, first motion data representing motion due to heartbeat may be acquired based on the first partial data 917. The first partial data 917 may be 1D data represented by lines. For convenience, referring to FIG. 9A, a phase value of the first partial data 917 may be indicated using a color included within a quadrangular area. In detail, a change in phase of pieces of projection data corresponding to the first partial data 917 may be obtained. Accordingly, first motion data representing motion due to heartbeat may be acquired.

Furthermore, second phase signal data corresponding to a boundary of the abdomen or chest may be acquired based on second partial data 915 in the projection data 913. Furthermore, second motion data representing motion due to respiration may be acquired based on the second partial data 915. The second partial data 915 may be 1D data represented by lines. For convenience, referring to FIG. 9A, a phase value of the second partial data 915 may be indicated using a color included within a quadrangular area. In detail, a phase correlation between pieces of projection data corresponding to the first partial data 915 may be obtained. Accordingly, second motion data representing motion due to respiration may be acquire.

FIG. 9B illustrates a first image 920 generated based on motion data according to an exemplary embodiment.

The first image 920 may include a first motion image 923 showing motion due to heartbeat and a second motion image 921 showing motion due to respiration.

The first and second motion images 923 and 921 in the first image 920 may have the same time axis 922. In addition, the first motion image 923 may be obtained by applying a high-pass filter to first motion data.

FIG. 9C illustrates a third motion image 930 according to an exemplary embodiment.

Referring to FIG. 9C, the third motion image 930 may include first motion data acquired during a first time interval (927 of FIG. 9B). In detail, the third motion image 930 may show one cardiac cycle for the object. At an R peak 935, a largest motion due to the heartbeat of the object may occur. Before or after a T peak 937 subsequent to the R peak 935, a small motion due to the heartbeat of the object may occur. According to an exemplary embodiment, to minimize degradation in image quality due to motion of the object, an MR image may be obtained during a second time interval 933 after the T peak 937.

In addition, a time interval (945 of FIG. 9B) may be determined based first motion data shown in the first motion image (923 of FIG. 9B) and second motion data shown in the second motion image (921 of FIG. 9B). By acquiring MRI data during the time interval 945, the user may obtain an MR image that suffers minimized degradation in quality due to reduced motion of the object.

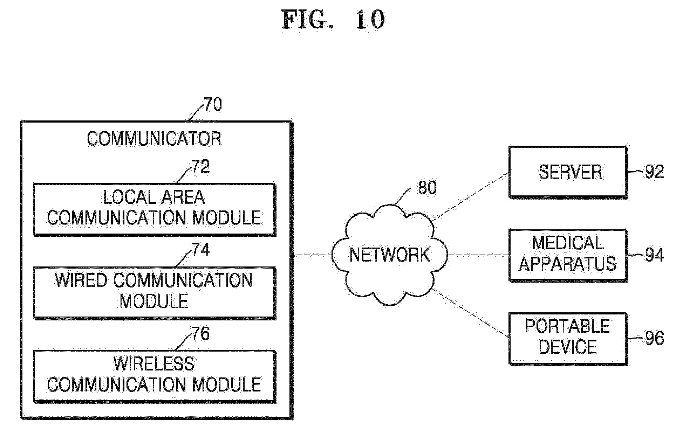

FIG. 10 is a block diagram of a communicator 70 according to an exemplary embodiment of the present disclosure.

Referring to FIG. 10, the communicator 70 may be connected to at least one selected from the gantry 20, the signal transceiver 30, the monitor 40, the system controller 50, and the operator 60 of FIG. 1.

The communicator 70 may transmit and receive data to and from a hospital server or another medical apparatus in a hospital, which is connected through a picture archiving and communication system (PACS), and perform data communication according to the digital imaging and communications in medicine (DICOM) standard.

As shown in FIG. 10, the communicator 70 may be connected to a network 80 by wire or wirelessly to communicate with a server 92, a medical apparatus 94, or a portable device 96.

In detail, the communicator 70 may transmit and receive data related to the diagnosis of an object through the network 80, and may also transmit and receive a medical image captured by the medical apparatus 94, such as a CT apparatus, an MRI apparatus, or an X-ray apparatus. In addition, the communicator 70 may receive a diagnosis history or a treatment schedule of the object from the server 92 and use the same to diagnose the object. The communicator 70 may perform data communication not only with the server 92 or the medical apparatus 94 in a hospital, but also with the portable device 96, such as a mobile phone, a personal digital assistant (PDA), or a laptop of a doctor or patient.

Also, the communicator 70 may transmit information about a malfunction of the MRI system or about a medical image quality to a user through the network 80, and receive a feedback regarding the information from the user.

The communicator 70 may include at least one component enabling communication with an external apparatus.

For example, the communicator 70 may include a local area communication module 72, a wired communication module 74, and a wireless communication module 76. The local area communication module 72 refers to a module for performing local area communication with an apparatus within a predetermined distance. Examples of local area communication technology according to an exemplary embodiment of the present disclosure include, but are not limited to, a wireless local area network (LAN), Wi-Fi, Bluetooth, ZigBee, Wi-Fi direct (WFD), ultra wideband (UWB), infrared data association (IrDA), Bluetooth low energy (BLE), and near field communication (NFC).

The wired communication module 74 refers to a module for performing communication by using an electric signal or an optical signal. Examples of wired communication technology according to an exemplary embodiment of the present disclosure include wired communication techniques using a pair cable, a coaxial cable, and an optical fiber cable, and other well known wired communication techniques.

The wireless communication module 76 transmits and receives a wireless signal to and from at least one selected from a base station, an external apparatus, and a server in a mobile communication network. Here, the wireless signal may be a voice call signal, a video call signal, or data in any one of various formats according to transmission and reception of a text/multimedia message.

The MRI apparatuses 200 and 300 of FIGS. 2 and 3 may be an external server 92, an external medical apparatus 94, or an external portable device 96. In other words, the MRI apparatuses 200 and 300 may be connected to the communicator 70 described with reference to FIG. 1 to be operated.

The exemplary embodiments may be written as computer programs and may be implemented in general-use digital computers that execute the programs using a non-transitory computer-readable recording medium.

Examples of the non-transitory computer-readable recording medium include magnetic storage media (e.g., ROM, floppy disks, hard disks, etc.), optical recording media (e.g., CD-ROMs, or DVDs), etc.

While one or more exemplary embodiments have been described with reference to the figures, it will be understood by those of ordinary skill in the art that various changes in form and details may be made therein without departing from the spirit and scope of the present disclosure as defined by the following claims. Accordingly, the above exemplary embodiments and all aspects thereof are examples only and are not limiting.

**Claims**

1. A magnetic resonance imaging (MRI) apparatus comprising:

    a signal transceiver;

a controller configured to control the signal transceiver to apply a gradient magnetic field in a blood flow direction, the blood flow direction being a direction in which a blood vessel is disposed within an object; and
an image processor configured to

receive, from the signal transceiver, phase signal data obtained by applying the gradient magnetic field to the object,
acquire motion data representing a first motion corresponding to a heartbeat of the object based on the phase signal data, and
acquire MRI data based on the motion data.

2. The MRI apparatus of claim 1, wherein the gradient magnetic field is a bipolar gradient magnetic field.

3. The MRI apparatus of claim 1 or 2, wherein the image processor is further configured to acquire motion data representing a second motion corresponding to a respiration of the object based on the phase signal data.

4. The MRI apparatus of claim 1, 2 or 3, wherein the image processor is further configured to acquire projection data with respect to a first cross-section showing the blood vessel and a boundary of an abdomen of the object and to acquire the phase signal data based on the projection data.

5. The MRI apparatus of claim 4, wherein the image processor is further configured to acquire the MRI data with respect to a second cross-section different from the first cross-section.

6. The MRI apparatus of claim 4 or 5, wherein the image processor is further configured to acquire first motion data representing the first motion based on first phase signal data corresponding to the blood vessel, and to acquire second motion data representing the second motion based on second phase signal data corresponding to a boundary of an abdomen.

7. The MRI apparatus of claim 5 or 6, wherein the image processor is further configured to acquire the MRI data during a time interval determined based on the first motion data and the second motion data.

8. The MRI apparatus of any of claims 1-7, wherein the image processor is further configured to acquire the MRI data using a first readout gradient and acquire the motion data using a second readout gradient, the second readout gradient being different from the first readout gradient.

9. The MRI apparatus of any of claims 1-8, further comprising a display configured to display at least one from among a first image generated based on the motion data and a second image generated based on the MRI data.

10. The MRI apparatus of any of claims 6-9, wherein the image processor is further configured to apply a high-pass filter to the first motion data.

11. A method of processing a magnetic resonance (MR) image, the method comprising:

controlling a gradient magnetic field to be applied in an blood flow direction, the blood flow direction being a direction in which a blood vessel is disposed within an object;
acquiring, based on phase signal data obtained by applying the gradient magnetic field to the object, motion data representing a first motion corresponding to a heartbeat of the object; and
acquiring magnetic resonance imaging (MRI) data based on the motion data.

12. The method of claim 11, wherein the gradient magnetic field is a bipolar gradient magnetic field.

13. The method of claim 11 or 12, further comprising acquiring, based on phase signal data obtained by applying the gradient magnetic field to the abject, motion data representing a second motion corresponding to a respiration of the object.

14. The method of claim 11, 12 or 13, wherein the acquiring of the motion data comprises:

acquiring projection data with respect to a first cross-section showing the blood vesseland a boundary of an abdomen of the object by applying the bipolar gradient magnetic field to the object; and
acquiring the phase signal data based on the projection data.

15. The method of claim 14, wherein the acquiring of the MRI data comprises acquiring, based on the motion data, the MRI data with respect to a second cross-section different from the first cross-section.

FIG. 1

OPERATOR 60

INPUT INTERFACE 66

OUTPUT INTERFACE 64

IMAGE PROCESSOR 62

MONITOR 40

SYSTEM MONITOR 42

OBJECT MONITOR 44

TABLE CONTROLLER 46

DISPLAY CONTROLLER 48

SYSTEM CONTROL UNIT 50

GANTRY CONTROLLER 58

SEQUENCE CONTROLLER 52

GRADIENT MAGNETIC FIELD CONTROLLER 54

RF CONTROLLER 56

SIGNAL TRANSCEIVER 30

GRADIENT AMPLIFIER 32

RF TRANSMITTER 36

RF RECEIVER 38

TRANSMISSION AND RECEPTION SWITCH 34

20  22  24  26  29  26  10  28

EP 3 103 389 A1

EP 3 103 389 A1

FIG. 2

200

CONTROLLER — 210

220

IMAGE PROCESSOR

222

MOTION DATA ACQUIRER

228

MRI DATA ACQUIRER'P

16

# FIG. 3

300

CONTROLLER 310

GRADIENT MAGNETIC FIELD CONTROLLER 312

SIGNAL TRANSCEIVER 340

IMAGE PROCESSOR 320

MOTION DATA ACQUIRER 322

MRI DATA ACQUIRER 328

DISPLAY 330

# FIG. 4

| CONTROL BIPOLAR GRADIENT MAGNETIC FIELD TO BE APPLIED | — S110 |

| ACQUIRE MOTION DATA REPRESENTING MOTIONS DUE TO HEARTBEAT AND RESPIRATION OF OBJECT | — S120 |

| ACQUIRE MRI DATA BASED ON MOTION DATA | — S130 |

# FIG. 5

FIG. 6

# FIG. 7A

## FIG. 7B

# FIG. 7C

# FIG. 8

ACQUIRE DC LINE IN K-SPACE — S210

PERFORM 1D INVERSE FOURIER TRANSFORM — S220

ACQUIRE PROJECTION DATA — S230

S240
ACQUIRE FIRST MOTION DATA REPRESENTING MOTION DUE TO HEARBEAT

S250
ACQUIRE SECOND MOTION DATA REPRESENTING MOTION DUE TO RESPIRATION

## FIG. 9A

FIG. 9B

FIG. 9C

# FIG. 10

COMMUNICATOR /70

LOCAL AREA COMMUNICATION MODULE /72

WIRED COMMUNICATION MODULE /74

WIRELESS COMMUNICATION MODULE /76

NETWORK /80

SERVER —92

MEDICAL APPARATUS —94

PORTABLE DEVICE —96

Europäisches
Patentamt

European
Patent Office

Office européen
des brevets

# EUROPEAN SEARCH REPORT

Application Number

EP 16 17 1530

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/097565 A1 (BASHA TAMER [US] ET AL) 9 April 2015 (2015-04-09) * abstract * * paragraphs [0014], [0022], [0071] - [0077], [0093], [0098] * | 1-15 | INV. A61B5/055 A61B5/11 G01R33/20 |
| X | EP 2 495 580 A1 (WISCONSIN ALUMNI RES FOUND [US]) 5 September 2012 (2012-09-05) * paragraphs [0012], [0039], [0050], [0060], [0061] * * figure 6 * | 1,11 | ADD. A61B5/113 G01R33/483 G01R33/565 |
| X | US 2013/053685 A1 (TAKEI NAOYUKI [JP]) 28 February 2013 (2013-02-28) * paragraphs [0003] - [0007], [0027], [0073], [0074] * | 1,11 | |
| X | US 2013/034287 A1 (ITAGAKI HIROYUKI [JP] ET AL) 7 February 2013 (2013-02-07) * paragraphs [0050] - [0064] * | 1,11 | |

TECHNICAL FIELDS
SEARCHED (IPC)

A61B
G01R

The present search report has been drawn up for all claims

| Place of search | Date of completion of the search | Examiner |
|---|---|---|
| The Hague | 9 November 2016 | Kowalczyk, Szczepan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 16 17 1530

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2016

| Patent document cited in search report | | Publication date | Patent family member(s) | | | Publication date |
|---|---|---|---|---|---|---|
| US 2015097565 | A1 | 09-04-2015 | KR | 20150039581 | A | 10-04-2015 |
| | | | US | 2015097565 | A1 | 09-04-2015 |
| EP 2495580 | A1 | 05-09-2012 | EP | 1927007 | A1 | 04-06-2008 |
| | | | EP | 1927009 | A1 | 04-06-2008 |
| | | | EP | 2495580 | A1 | 05-09-2012 |
| | | | JP | 5113060 | B2 | 09-01-2013 |
| | | | JP | 5113062 | B2 | 09-01-2013 |
| | | | JP | 2009508634 | A | 05-03-2009 |
| | | | JP | 2009508655 | A | 05-03-2009 |
| | | | US | 2007106149 | A1 | 10-05-2007 |
| | | | US | 2007167707 | A1 | 19-07-2007 |
| | | | WO | 2007037937 | A1 | 05-04-2007 |
| | | | WO | 2007038205 | A1 | 05-04-2007 |
| US 2013053685 | A1 | 28-02-2013 | JP | 2013048780 | A | 14-03-2013 |
| | | | US | 2013053685 | A1 | 28-02-2013 |
| US 2013034287 | A1 | 07-02-2013 | JP | 5815508 | B2 | 17-11-2015 |
| | | | US | 2013034287 | A1 | 07-02-2013 |
| | | | WO | 2011132593 | A1 | 27-10-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020150073923